# EUROPEAN PATENT APPLICATION

(11) **EP 1 252 897 A1**
(43) Date of publication of application: **30.10.2002**
(21) Application number: 01901470.3
(22) Date of filing: 19.01.2001
(51) Int. Cl.: A61K 48/00, A61K 38/44, A61K 31/711, A61K 35/76

(54) **DRUGS FOR GENE THERAPY**

(30) Priority: 21.01.2000 JP 2000050324
(71) Applicant: HISAMITSU PHARMACEUTICAL CO., INC., Tosu-shi Saga 841-0017 (JP)
(72) Inventor: YUDA, Kazuhiro c/o Tsukuba Laboratory, Tsukuba-shi, Ibaraki 305-0856 (JP); MAEKAWA, Koutarou c/o Tsukuba Laboratory, Tsukuba-shi, Ibaraki 305-0856 (JP); AKIYAMA, Katsuhiko c/o Tsukuba Laboratory, Tsukuba-shi, Ibaraki 305-0856 (JP); GOTO, Takeshi c/o Tsukuba Laboratory, Tsukuba-shi, Ibaraki 305-0856 (JP); GOTO, Sigeru, Niao-Sung (TW)
(74) Representative: Grünecker, Kinkeldey, Stockmair & Schwanhäusser Anwaltssozietät
(86) International application number: JP0100353
(87) International publication number: WO01052901

(57) **Abstract**

This invention relates to a gene therapeutic agent comprising indoleamine 2,3-dioxygenase (IDO) and to an immunosuppresant comprising an IDO protein, both of which are particularly effective for rejection in organ transplantation. Further, it is possible to suppress the rejection more effectively by forming a complex between the IDO gene and a gene carrier.

## Description

### Technical Field

This invention relates to a gene therapeutic agent comprising an indoleamine 2,3-dioxygenase (IDO) gene and a gene carrier and to an immunosuppresant (or an immunosuppressive agent) containing an IDO protein.

### Background Art

Nowadays, since surgical technologies for organ transplantation have advanced remarkably, the success of organ transplantation depends on how the transplant rejection following the surgery can be controlled. When the living body recognizes the graft as a foreign substance, it induces a series of immunological reactions to eliminate the graft, which result in the rejection. The immunosuppresants that have been clinically used to suppress these immunological reactions include steroids (such as prednisolone), antimetabolites (such as azathiopurine and mizoribine), and antibiotics (such as cyclosporin A and tacrolimus). With the advent of these drugs, kidney, heart or liver transplantation has already been recognized as general medical treatment and lung, pancreas, or small intestine transplantation has gradually come into wider use because of improved results ("Today's Transplantation," Vol. 11, No. 1, 53, 1998).

However, these drugs have side effects in no small degrees. For example, steroids have wider action as compared with other immunosuppresants, but their immunosuppressing action is generally weak and a decrease in their effectiveness or the manifestation of resistance may be seen over continuous administration for a long period of time. Azathiopurine exerts an immunosuppressive effect by inhibiting nucleic acid synthesis, but its use may increase the risk of developing an infection, e.g., by bacteria as well as may cause bone marrow suppression (a fall in the leukocyte count, a decrease in platelet, and anemia) and a liver disorder. When mizoribine is compared with azathiopurine, mizoribine has the advantage of fewer cases of bone marrow suppression and liver disorders; on the other hand, its immunosuppressing effect is not so great. Cyclosporin A and tacrolimus have very potent immunosuppressive activity and are the drugs that have contributed to improved results of organ transplantation.

However, it has been shown that these drugs have serious central nervous toxicity, kidney toxicity and the like. Close attention will thus be needed for the use of the drugs ("Today's Transplantation," Vol. 11, No. 1, 37, 1998).

Liver is catching attention as a very unique organ in organ transplantation. This is because the sympatric liver transplantation has been shown to be successful without administration of any immunosuppresant in a combination of certain animal species (Nature, Vol. 223, 427, 1969). Independently, it was thought that a disabled liver or a regenerating liver might produce immunosuppressing substances since immunosuppressive conditions prevail in the disorder of hepatic cells such as hepatitis (Nature, Vol. 251, 655, 1974).

Under these circumstances, several kinds of immunosuppressive proteins have been reported: their representatives are described below.

### (L-Arginase)

L-Arginase is an immunosuppressive protein isolated from a hepatic cell and has been thought to be released in blood when the liver is disabled. It is understood that once released in a large quantity, L-arginase digests L-arginine to form L-ortinine and urea and to generate an arginine deficient state in the body whereby the rapid proliferation of lymphocytes is suppressed. (J. Immunol., Vol. 131, 2427, 1983; Japanese Patent Application Laid-Open Gazette No. Hei 3-157399.) However, this state can be improved by adding arginine; hence, there is a question as to whether rejection can be suppressed over a long period of time in the living body where the amounts of arginine supplied differ among individual cells, for example in the liver having the great capability of supplying arginine. Digestion of arginine in a large quantity also produces a large quantity of urea at the same time. This will cause hyperazotemia which is clinically problematic, and the possibility is suggested that kidney failure may be caused (Medicina, Vol. 31, No. 11, 60, 1994).

### (Very Low Density Lipoprotein (VLDL))

It is thought that this substance is secreted from hepatic cells and manifests in serum. VLDL inhibits DNA synthesis of peripheral blood monocytes and suppresses their activation (J. Immunolo., Vol. 119, 2129, 1977). However, when VLDL is administered as a drug for a prolonged period, it chronically increases serum VLPL and the levels of intermediate density lipoprotein (IDL) and low density lipoprotein (LDL) that have been converted from VLDL by lipoprotein lipase, which possibly causes hyperlipemia, triglyceride blood disease, diabetes, uremia, and arteriosclerosis (Medicina, Vol. 31, No. 11, 179, 1994). (α―Fetoprotein and Early Pregnancy Factor)

It is also known that immunosuppressive proteins are secreted from the liver in a special state. α ― Fetoprotein, which is regarded as a tumor maker for hepatic carcinoma, is provided with the immunosuppressive power; and it has been identified from embryonic mice and investigated (J. Exp. Med., Vol. 141, 269, 1975).

Early pregnancy factor is a protein that has been detected in the urine of a woman in the early phase of pregnancy and that has the immunosuppressive power. It has also been confirmed that the protein is secreted after the hepatic lobectomy (Nature, Vol. 278, 649, 1979).

These proteins are the substances that suppress the proliferation of lymphocytes in a non-specific manner and are regarded as the substances that are involved in the vivid regeneration power of liver when the liver has been disabled. However, this has failed to account for the immune tolerance induced by liver transplantation.

### (Soluble Class I Antigen)

A large quantity of the soluble class I antigen of the donor type is observed in the blood of the recipient immediately after transplantation. It has been thought that the substance induces immune tolerance, but immunosuppression is not adequately induced even upon administration of the substance (Transplantation, Vol. 50, 678, 1990).

### (Liver Suppressor Factor-1 (LSF-1))

It is an immunosuppressive protein that has been obtained by collecting the serum from the rat after liver transplantation in a time-dependant manner and by subjecting the serum to electrophoresis on SDS-PAGE. Its action is estimated to be very high, but the protein has not been identified (Transplant. Immunol., Vol. 3, 174, 1995). Further studies will then be necessary to put it in practical use as a drug.

Apart from those mentioned above, concerning the growth of conceptus from murine parents of different lines, Munn et al. showed that IDO was expressed in the polynuclear trophoblast layer of a placenta and that it suppressed the rejection of the conceptus: IDO is an immunosuppressive protein derived from an organ other than liver. (Science, Vol. 281, 1191, 1998.) It has also been confirmed that IDO is expressed in macrophages or the like and that it suppresses the proliferation of T cells (J. Exp. Med., Vol. 189, 1363, 1999; WO99/29310).

However, the immunosuppresants that are currently used clinically are known to possess side effects such bone marrow suppression, liver damage, central nerve toxicity, and renal toxicity; and there has not been found any substance that is an immunosuppressive protein derived from the liver and that can be clinically utilized. In addition, there have not been specifically disclosed dosage forms or methods of administration as an immunosuppresant for IDO.

### Disclosure of the Invention

It is an object of this invention to provide an immunosuppresant that is effective for the prevention of rejection in organ transplantation as well as for the treatment of an autoimmune disease or an allergic disease and that can be used without side effects.

As a result of having pursued diligent studies to solve the above-stated problems, the present inventors found that the expression level of the IDO gene increased in the rat liver after liver transplantation.

Further, the present inventors prepared a gene therapeutic agent for expressing an IDO gene and found that when this agent was used in the sympatric liver transplantation, using rats of different lines, which produced rejection, the rejection could, as a result, be effectively suppressed.

Specifically, this invention provides a gene therapeutic agent comprising a gene encoding IDO. The invention also provides a gene therapeutic agent comprising a gene carrier in addition to the gene therapeutic agent. The gene carrier is characterized by being a viral vector or a cationic gene carrier. As the cationic gene carrier there are mentioned cationic polyamino acids (such as polylysine and polydiaminobutyric acid) and cationic synthetic polymers (such as liposome and ethylenimine polymer). In the case of a cationic polyamino acid, a gene carrier comprising diaminobutyric acid or a pharmaceutically acceptable salt thereof is preferable; and in the case of a cationic synthetic polymer, ethylenimine polymer is preferable. This invention also provides an immunosuppresant comprising an IDO protein as the effective ingredient.

The gene therapeutic agent comprising the IDO gene and the IDO protein according to this invention are useful in the prevention of rejection in organ or tissue transplantation and are effective as therapeutics for autoimmune diseases and inflammatory diseases such as allergic disorders.

### Brief Description of the Drawings

Fig. 1 is a graph showing the difference in the expression capability of the IDO gene between non-treated rats and the rats having undergone sympatric liver transplantation. In the figure, "DA" represents a DA rat, "PVG" represents a PVG rat, "DA/DA" represents a rat having undergone the sympatric liver transplantation between the DA rats, and "DA/PVG" represents a rat having undergone the sympatric liver transplantation from the DA rat to the PVG rat.

Fig. 2 is a graph showing the immunosuppressive activity of the IDO protein in a lymphocyte mix culture test.

### Best Mode for Carrying Out the Invention

The gene therapeutic agent for expressing the murine IDO gene and the murine IDO according to this invention may be prepared by the technique shown below.

Clones of the IDO gene can be obtained by screening a suitable cDNA for which the expression of the IDO gene has been confirmed and which is commercially available, including those derived from a placenta at full term normal parturition and a lung. For the screening method, there are techniques known in the art such as PCR and plaque (colony) hybridization.

The IDO gene contained in the gene therapeutic agent of this invention is shown as the sequence set forth in SEQ ID NO:1, and the therapeutic agents of this invention also encompass the genes capable of expressing an IDO possessing substantially the same IDO activity as does the IDO gene. As used herein, the expression, "substantially the same" means that the particular sequence of interest differs from the reference sequence by one or more substitutions, deletions, or additions, but as a final result, it possesses the biological activity of the IDO protein.

The gene therapeutic agent of this invention may be constructed by inserting the IDO gene into an expression plasmid for eucaryotic cells or into a viral vector.

The expression plasmids for eucaryotic cells include plasmids having the replication origin of a simian virus 40 (SV40), an Epstein Barr virus (EBV), or a bovine papilloma virus (BPV), and plasmids having a SV40 promoter or a cytomegalovirus (CMV) promoter: for the latter there are specifically mentioned plasmids such as pBK-CMV (Stratagene), pZeoSV2(+) (Invitrogen Corporation), pCMV · SPORT (GIBCO), and pcDNA3.1+ (Invitrogen Corporation). The viral vectors include a murine leukemia virus vector, an Adenovirus vector, an Adeno-associated virus vector, a human immunodeficiency virus (HIV) vector, a herpes simplex virus vector, and a Sendai virus vector. The method mentioned above is known and a procedure therefor is introduced in many experiment texts such as "Laboratory Manuals: Gene Manipulation of Animal Cells" 30 (1994) published by Takara Shuzo Co., Ltd.

When the gene therapeutic agent of this invention is administered to human, therapeutic effects can be expected from the IDO gene originating in any species, but preferably it contains the human IDO gene (SEQ ID NO:1) as the effective ingredient because of the problem of immunogenicity. It is sufficient that the human IDO gene contains at least the whole length for the region encoding the protein. Further, when the IDO protein is administered to human, the protein of the human type is preferably administered.

The gene therapeutic agent of this invention may be prepared as a complex between the IDO gene and a gene carrier. Therapeutic effects can be expected for either of the viral vector and the cationic gene carrier. The viral vectors, for example, include a murine leukemia virus vector, an Adenovirus vector, an Adeno-associated virus vector, a HIV vector, a herpes simplex virus vector, and a Sendai virus vector. The Adeno-associated virus vector is preferably used.

The cationic gene carriers, for example, include the substances that have affinity to genes: polyamino acids (such as polylysine and polydiaminobutyric acid) and cationic synthetic polymers (such as liposome and ethylenimine). Particularly, in order to suppress rejection in liver transplantation, polydiaminobutyric acid is preferably used that has been known for its high degree of migration to the liver.

When referred to as "polydiaminobutyric acid" in this invention, it is a polypeptide comprising diaminobutyric acid or a polypeptide comprising a pharmaceutically acceptable salt thereof. Alternatively, it may be a structure that contains these two structures (diaminobutyric acid and its pharmaceutically acceptable salt) in any proportion. Furthermore, while optical isomers, L-form and D-form, exist in diaminobutyric acid, the polydiaminobutyric acid in the present specification is a polypeptide containing D-form, L-form or both of them in any proportion. The number of diaminobutyric acid contained in one molecule of polydiaminobutyric acid in the present specification is at least 10 residue (in the case of polyaminobutyric acid acetate, its molecular weight is 1,600 or greater), preferably 20 or more residues (in the case of polyaminobutyric acid acetate, its molecular weight is 3,200 or greater), and more preferably 25 or more residues (in the case of polyaminobutyric acid acetate, its molecular weight is 4,000 or greater) from the standpoint of the performance as the gene carrier. In addition, the number is preferably 280 residues or less (in the case of polyaminobutyric acid acetate, its molecular weight is 44,800 or less), and more preferably 250 residues or less (in the case of polyaminobutyric acid acetate, its molecular weight is 40,000 or less) in view of the difficulty of synthesis and the inconvenience of handling.

The salts of diaminobutyric acid are not particularly limited insofar as they are pharmaceutically acceptable salts. There are mentioned, among others, the salts of inorganic acid such as hydrochloric acid, sulfuric acid, nitric acid, and phosphoric acid and the salts of organic acid such as acetic acid, propionic acid, citric acid, lactic acid, oxalic acid, succinic acid, tartaric acid, malonic acid, fumaric acid, and malic acid. Acetates are particularly preferred.

There is no particular limitation to the method for synthesizing the polydiaminobutyric acid mentioned above and there can be used organic chemical reactions including a variety of synthetic methods for polypeptides that are known in the art (New Experimental Chemistry Monograph No. 19 Polymer Chemistry I, pp. 244 (1980), Maruzen Co., Ltd.). More specifically, monomer components can be polymerized through a suitable reaction to produce polydiaminobutyric acid. In this case, the monomers to be used may preferably employ diaminobutyric acid, diaminobutyric acid where a protective group has been introduced into the γ-amino group, and diaminobutyric acid having activated amino groups and/or carboxylic acid groups for peptide group formation. Especially, it is preferred that the γ-amino group be protected with a suitable protecting group and the amino acid portion be converted, using phosgene, into an acid anhydride in order to render the peptide bond formation easy. In order to polymerize such monomers, it is possible to use various kinds of initiators in appropriate quantities. Concretely, various amine and alcohol compounds are usable. The amine compounds include alkylamines; particularly, butylamine is preferable.

In preparing a complex, the composition ratio of the IDO encoding gene to polydiaminobutyric acid is preferably from 1/0.1 to 1/100 (W/W ratio) from the standpoint of efficiency of gene transfer. If the composition ratio of gene to polydiaminobutyric acid is 1/0.1 or less, sufficient therapeutic effects cannot be expected; further, if the composition ratio is 1/100 or more, the content of the gene carrier is too much and the free gene carrier that does not participate in complex formation increases with the tendency to cause cell damage, which is not preferred.

In the case of a straight-chain polymer of ethylenimine such as ExGen 500 (Wako Pure Chemical and Industries, Ltd.), similar effects as those with polydiaminobutyric acid can be expected. In preparing a complex, the composition ratio of the IDO encoding gene to ExGen 500 is preferably from 1/0.5 to 1/7 (W/V ratio) from the standpoint of efficiency of gene transfer. If the composition ratio of gene to ExGen 500 is 1/0.5 or less, there is a tendency that sufficient therapeutic effects cannot be expected; conversely, if the composition ratio is 1/7 or more, there is a tendency that sufficient therapeutic effects cannot be expected, which is not preferred. Furthermore, it will be possible to carry out administration through a plasmid containing the IDO gene without using the gene carrier mentioned above provided that sufficient therapeutic effects can be obtained. Gene therapy can be carried out *ex vivo* or *in vivo* using these IDO genes.

The IDO protein can be purified from an organ by carrying out manipulations according to the method of Yamazaki et al. (Wakayama Med. Soc., Vol. 38, No. 4, 447, 1987). Specifically, the enzyme is extracted from the organ expressing IDO (e.g., placenta) with 0.25 M sucrose solution; a supernatant from centrifugation at 10,000 g for 30 minutes is used to prepare a crude extract; column purification is carried out at seven stages of phospho-cellulose, hydroxyapatite, Sephadex G-100, isoelectric focusing, CM Affi-Gel Blue, TSK CM-3, and SW; and thereby purification of the IDO protein is possible.

The IDO protein can also be prepared by genetic manipulation techniques. The murine IDO gene sequence (Gene, 105, 221-227, (1991): Accession No. M69109) and the human IDO gene sequence (Biochem. Biophys. Res. Commun., 168, 1 (1990): Accession No. M34455) are known. Thus, probes or PCR primers are constructed based on these kinds of genetic information, and cDNA libraries or RNAs prepared from cells or organs are used to clone the IDO gene according to colony (plaque) hybridization or (RT-) PCR either of which is a technique known in the art. An IDO expression vector can be constructed by inserting the gene cloned by the aforementioned technique into an expression plasmid for *E. coli* such as pBluescript SK(+). The expression as a fusion protein with the GST protein can be done to facilitate the purification insofar as the activity of the IDO protein may be retained. Such an IDO expression plasmid is introduced into *E. coli*, and after large-scale culturing, cells are recovered: by carrying out the manipulations according to the method of Maeda (Wakayama Med. Soc., Vol. 44, No. 3, 401, 1993), it is possible to purify the recombinant IDO protein having the same physiological activity as that of naturally occurring IDO. Specifically, there can be used the variants of the IDO protein where one amino acid, or two or more amino acids are substituted, added or deleted in order to enhance its immunosuppressive activity, to improve its stability in blood, to reduce its antigenicity, and/or to ameliorate its side effects. Such IDO protein variants can be prepared by constructing variant IDO gene sequences corresponding to one amino acid, or two or more amino acids to be substituted, added or deleted. Further, such IDO protein variants can be produced by introducing plasmids having the variant IDO gene sequence into suitable microorganisms or animal cells.

The agents comprising the IDO protein as the effective ingredient are not particularly limited, and they can be modified with suitable substances as long as the substances are pharmaceutically acceptable. Specifically, the modification is possible as long as it does not lower the activity of said protein, nor gives undesirable toxicity to the living body. For example, the modifications with poly(ethylene glycol) or dextran are effective for prolonging half-life in blood.

The present inventors first studied the immunosuppressive effect of the IDO protein. A lymphocyte mix culture test was conducted, and as a result, the immunosuppressive effect was observed in the group with addition of the IDO protein. The present inventors next studied the rejection-suppressing effect using the gene therapeutic agent containing the IDO gene. When the sympatric liver transplantation was carried out from a DA rat to a LEW rat (a combination where an organ graft is to be rejected), a marked increase in the days of take was observed in the group where the IDO expression plasmid was administered as the effective ingredient to the liver. ("Inflammation and Immunity," Vol. 4, No. 3, 28, 1996, Immunol. Today, Vol. 6, 336, 1985.) Furthermore, to ascertain that the IDO protein is involved in the suppression of the rejection in transplantation, the present inventors administered an inhibitor of the IDO protein to the sympatric liver transplantation model of a DA rat to a PVG rat for which a liver graft was known to be taken without the use of an immunosuppresant (SURGERY, Vol. 93, No. 1, 64, 1983). Consequently, in the group with the administration of 1-methyl-tryptophan which is an IDO inhibitor, it was found that the liver graft was not taken.

Therefore, the gene therapeutic agent comprising the IDO gene and the medicament comprising the IDO protein as the effective ingredient are effective immunosuppresants and are particularly effective against rejections in the transplantation of organs or tissues such as heart, liver, lung, spleen, kidney, small intestine, skin, or bone marrow. They are also effective as therapeutics for autoimmune diseases such as rheumatism, atopy, and systemic lupus erythematosus as well as for inflammatory diseases such as allergic disorders, e.g., pollinosis. Single or repetitive administration effectively suppresses the rejection.

The gene therapeutic agent comprising the IDO gene and the medicament comprising the IDO protein as the effective ingredient according to this invention may be administered alone, or may be administered in the forms combined with pharmaceutically acceptable substances. For example, in the case of injection, the IDO gene or the IDO protein of this invention can be dissolved in water to prepare it. As necessary, it may be prepared by dissolving the gene or the protein in physiological saline, glucose solution or the like, and it may contain a buffer, a preservative, or a stabilizer. These preparations may also contain other ingredients that are of value therapeutically.

With respect of the method of administration for the immunosuppresant of this invention, oral administration, injection, intrarectal administration, intraportal administration, perfusion of an organ graft, local administration to the organ graft, and the administration utilizing a balloon catheter are possible when the rejection at the time of transplantation is to be treated. In administering the IDO gene to suppress the rejection at the time of liver transplantation, administration through injection is preferred and intraportal administration is more preferred. The dosages differ depending on the method of administration, the conditions or the age of the patient. Where the IDO gene is administered, single administration or repetitive administration at the intervals of a few days to a few months may be normally done at 0.01-100 mg/kg, or preferably at 0.1-20 mg/kg (as the quantity of the IDO gene) per administration. Where the IDO protein is administered, the administration through injection is preferable and single administration or repetitive administration at the intervals of a few days to a few months may be done at 0.01-100 mg/kg, or preferably at 0.1-10 mg/kg (as the quantity of the IDO protein) per administration.

### EXAMPLES

This invention will be described more concretely by referring to the examples; however, the invention is not to be limited by the following examples.

### (Example 1) Measurement of transcription level of the IDO gene in a sympatric liver-grafted rat

concerning the expression capability of IDO, sympatric liver transplantation between different lines was conducted without using an immunosuppresant where the liver of a DA rat was grafted to a PVG rat (a combination where a liver graft was taken without the use of an immunosuppresant) and the correlation between the endogenous IDO and the "take" of the organ was studied.

The operation of transplantation was carried out using the technique of sympatric liver transplantation developed by Kamada et al. (SURGERY, Vol. 93, 64, 1983). On the 7th day after the operation livers were extracted from the rat having undergone the sympatric liver transplantation, the non-treated PVG rat, and the DA rat; the organs were rapidly frozen using liquid nitrogen, and then they were ground in pestles. One hundred milligrams was weighed and total RNA was prepared using TRI REAGENT (Molecular Research Inc.). RT-PCR was performed using a 1st Strand cDNA Synthesis Kit for RT-PCR (Beringer Manheim Corporation). In reverse transcription reaction, 100 ng of total RNA, 1xreaction, 5 mM MgCl₂, 1 mM dNTP, 50 units RNase inhibitor, 10 units AMV reverse transcriptase, and 1.6 µg oligo (dT)₁₅ were mixed and the volume was made 20 µl with DEPC-H₂O. Reaction was carried out at 25 ° C for 10 minutes, at 42 ° C for 1 hour, and at 95 ° C for 5 minutes. In PCR reaction, 1 µ l of the mixed solution after the reverse transcription reaction was used as a template and to each 0.1 µM of PCR primers of SEQ ID NO:2 and SEQ ID NO:3 (SAWADAY Technologies Co., Ltd.) were added 1.5 mM MgCl₂, 1 mM dNTP, and 2.5 units of Taq DNA Polymerase. The final volume was adjusted to 50 µ l with sterilized water. After incubation at 94 ° C for 2 minutes, a cycle of 94 ° C/30 seconds, 55 ° C /1 minute, and 68 ° C/2 minutes was repeated 30 times followed by incubation at 68 ° C for 10 minutes.

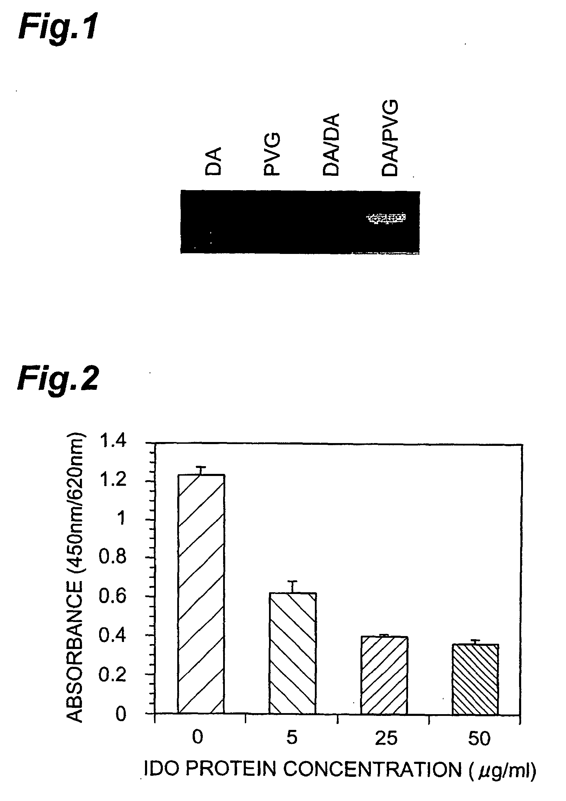

The mixed solution after the aforementioned reaction was analyzed by electrophoresis on 2.0% agarose gel. As a result, the transcription level of the IDO gene (about 740bp) was detected only in the group where the sympatric liver transplantation was carried out (Fig. 1). This demonstrated that one of the endogenous factors for suppressing the rejection of organ transplantation was IDO.

### (Preparation Example 1) Preparation of murine IDO gene

The murine IDO gene was prepared by PCR. Reaction was carried out similarly to Example 1, where 0.5 µg of Mouse Lung cDNA Library (Takara Shuzo Co. Ltd.) was used as the template in combination with PCR primers of SEQ ID NO:4 and SEQ ID NO:5 (SAWADAY Technologies Co., Ltd.).

The mixed solution after the reaction was subjected to electrophoresis using 1.0% agarose gel, and a PCR fragment of about 1.3 kb was recovered from the gel with a Geneclean II kit (Funakoshi Co., Ltd.).

### (Example 2) Construction of murine IDO expression plasmid

Plasmid (pcDNA3.1(+)), 0.5 µg, and the recovered PCR fragment were respectively subjected to the restriction enzyme treatment at 37 ° C overnight, using NheI (NEB) and XhoI (NEB). By allowing to migrate by electrophoresis on 0.8% agarose gel, the respective fragments after the restriction enzyme treatment were recovered using a Geneclean II kit. These were mixed at the ratio of pcDNA3.1(+), 0.1 µg, to the PCR fragment, 0.5 µg, and ligation reaction was carried out overnight using T4 DNA Ligase (GIBCO).

Competent cells, of *E. coil* JM109 (Takara Shuzo Co., Ltd.), 100 µl, were dissolved in ice and to this was added 5 µl of the reaction solution after the ligation, which was allowed to stand in ice for 30 minutes. After incubation at 42 ° C for 90 seconds, the cells were placed in ice again, and to this was added 900 µl of SOC medium (GIBCO), followed by further incubation at 37 ° C for 1 hour. One hundred microliters was seeded on a LB agar medium containing 50 µg/ml of ampicillin (Wako Pure Chemical Industries, Ltd.) and incubated at 37 ° C overnight. Then, colonies were picked up and added to a LB liquid medium containing 50 µg/ml of ampicillin; and further culturing was carried out at 37 ° C overnight. Purification of the plasmid was carried out using a Quiagen Plasmid MINI kit (QIUAGEN Corporation). The gene sequence of the obtained plasmid was analyzed with a DNA sequencer ALFexpress (Amersham Pharmacia Biotech Inc.) and was confirmed to be the normal IDO gene with no point mutation introduced. The aforementioned manipulations produced an expression plasmid (IDO/pcDNA) into which the IDO gene as shown in SEQ ID NO:1 had been inserted.

### (Example 3) Preparation of IDO/pcDNA/cationic gene carrier complex

The complex between IDO/pcDNA and a gene carrier was prepared. Preparation of the complex was as follows.

### (Preparation of IDO/pcDNA/ExGen complex)

A solution of ExGen 500 (Wako Pure Chemical Industries, Ltd.), a straight-chain polymer of ethylenimine, was used with the addition of five-fold physiological saline to prepare a carrier solution. Three kinds of IDO/pcDNA/ExGen complexes with differing composition ratios were prepared by mixing equal amounts of the IDO/pcDNA solution and the gene carrier solution and then by allowing them at room temperature for 10 minutes (plasmid/ExGen ratio=1/0.1, 1/1, and 1/10 w/v ratios). There were used ExGen 500 solution and IDO/pcDNA solution whose volumes and concentrations are shown in Table 1. The complex solutions were adjusted with physiological saline to their final volumes of 100 ml.

**Table 1**

| Preparation of IDO/pcDNA/ExGen Complexes | | | |
|---|---|---|---|
| plasmid/gene carrier (w/v ratio) | ExGen 500 solution | IDO/pcDNA solution | |
| | | Concentration | Volume |
| 1/0.1 | 100 µl | 2 mg/ml | 500 µl |
| 1/1 | 1 ml | 200 µg/ml | 5 ml |
| 1/10 | 10 ml | 20 µg/ml | 50 ml |

### (Preparation of IDO/pcDNA/polydiaminobutyric acid complexes)

A DMEM medium (Sigma-Aldrich Corporation) was used to prepare 20 ml of polydiaminobutyric acid (the average number of residues per molecule: 50) solutions (0.5, 250, and 25000 µg/ml). These solutions were added dropwise to 20 ml of the IDO/pcDNA solution (50 µg/ml) prepared with DMEM medium, and then, they were allowed to stand at room temperature for 30 minutes to prepare IDO/pcDNA/polydiaminobutyric acid complexes (plasmid/polydiaminobutyric acid weight ratio=1/0.01, 1/5 and 1/500).

### (Example 4) Construction of recombinant Adenovirus vector for expression of murine IDO

Construction of the Adenovirus vector was carried out using an Adenovirus Expression Vector Kit (Takara Shuzo Co., Ltd.). PCR primers of SEQ ID NO:6 and SEQ ID NO:7 were used to produce a PCR fragment similarly to Preparation Example 1. After this PCR fragment and pAxcw were subjected to the restriction enzyme treatment with Swa I (Takara Shuzo Co., Ltd.) according to a method similar to Example 2, the PCR fragment was inserted to pAxcw, producing IDO/pAxcw. The technique to follow for the preparation of the recombinant Adenovirus vector was carried out according to the method of Kanegafuchi et al. (Experimental Medicines (Special Issue), Vol. 12, No. 15, 1804, 1994; Cell engineering, Vol. 13, No. 8, 757, 1994; Experimental Medicines (Supplemental Issue), "New Genetic Engineering Handbook," 238, 1996; Experimental Medicines (Supplemental Issue), "Bio-manual Up Series, Basic Technologies in Gene Therapy," 91, 1996; Experimental Medicines (Supplemental Issue), "The Protocol Series, Experimental Method on Gene Transfer and Expression Analysis," 27, 1997.) Consequently, 3x10⁹ PFU/ml of the recombinant Adenovirus vector for expression of the murine IDO (IDO/Ad) was obtained.

### (Example 5) Purification of recombinant murine IDO protein

Employing the techniques similar to those of Preparation Example 1 and Example 2, pGEX-4T-2 (Amersham Pharmacia Biotech Inc.) and a PCR fragment obtained through the use of SEQ ID NO:8 and SEQ ID NO:9 were subjected to the restriction enzyme treatment with SmaI and NotI (NEB). The PCR fragment was then inserted to pGEX-4T-2 to produce a murine IDO protein expression plasmid (IDO/GEX). After gene transfer to *E. coli* JM109 with this plasmid, isopropyl- β -D-thiogalactopyranoside (Wako Pure Chemical Industries, Ltd.) was added to produce a final concentration of 0.1 mM, whereby the expression of the recombinant IDO protein was induced. Purification of this protein was carried out according to the method of Maeda (Wakayama Med. Soc., Vol. 44, No. 3, 401, 1993). Finally, about 12.5 mg of the recombinant murine IDO protein was obtained from 10 l of the *E. coli* culture solution.

### (Example 6) Studies on the immunosuppressive action by IDO gene administration using acute rejection model

To judge the therapeutic effect of IDO on the acute rejection after organ transplantation, the sympatric liver transplantation model of a DA rat to a LEW rat for which the acute rejection was reported to occur leading to death was administered with the IDO gene/cationic gene carrier complex prepared in Example 3 and with IDO/Ad prepared in Example 4, respectively. As for the animals, male LEW and DA rats (200-350 g) were used. In the administration of the aforementioned gene therapeutic agents, immediately after Ringer's Solution (Fuso Pharmaceutical Co. Ltd.) was used in the operation and was allowed to perfuse the portal veins and the liver, the respective agents were infused from the portal veins into the liver at a speed of 5 ml/min. After allowing to stand in the culture solution for 30 minutes, the liver into which the gene had been transferred was grafted to the LEW rat.

As the results are shown in Table 2, a marked increase in the days of take of the liver graft was observed in the group where IDO/pcDNA/ExGen was administered at the W/V ratio of 1/1, in the group where IDO/pcDNA/polydiaminobutyric acid was administered at the W/W ratio of 1/5, and in the group where IDO/Ad was administered at 1x10⁹ PFU/rat. As compared to the non-treated group, a slight increase in the days of take of the liver graft was observed in the group where IDO/pcDNA/ExGen was administered at the W/V ratio of 1/0.1, in the group where IDO/pcDNA/polydiaminobutyric acid was administered at the W/W ratio of 1/0.01, and in the group where IDO/Ad was administered at 1x10⁷ PFU/rat. Further, there was death at a stage earlier than the non-treated group in the days of take of the liver graft was observed in the group where IDO/pcDNA/ExGen was administered at the W/v ratio of 1/10 and in the group where IDO/pcDNA/polydiaminobutyric acid was administered at the W/W ratio of 1/500.

**Table 2**

| Assay of the immunosuppressive activity of the IDO protein using acute rejection model (N=6) | | | |
|---|---|---|---|
| recipient | donor | treatment | average days of take (survival) (day) |
| LEW | DA | non-treated | 11.5 |
| LEW | DA | IDO/pcDNA/ExGen W/V ratio=1/0.1 | 16.5 |
| LEW | DA | IDO/pcDNA/ExGen W/V ratio=1/1 | >30 |
| LEW | DA | IDO/pcDNA/ExGen W/V ratio=1/10 | 3.2 |
| LEW | DA | IDO/pcDNA/polydiaminobutyric acid W/W ratio=1/0.01 | 17.7 |
| LEW | DA | IDO/pcDNA/polydiaminobutyric acid W/W ratio=1/5 | >30 |
| LEW | DA | IDO/pcDNA/polydiaminobutyric acid W/W ratio=1/500 | 2.6 |
| LEW | DA | IDO/Ad 1x10⁹ PFU/rat | >30 |
| LEW | DA | IDO/Ad 1x10⁷ PFU/rat | 15.6 |

### (Example 7) Studies on the immunosuppressive activity of the IDO protein using acute rejection model

A model similar to that in Example 2 was used to judge the therapeutic effect of the IDO protein prepared in Example 5. The IDO protein was administered consecutive days through the tail vein immediately after the liver transplantation at a dosage of 300 µg/rat. As the results are shown in Table 3, a marked increase in the days of take of the liver graft was observed in the group where 300 µ g of the IDO protein was administered.

**Table 3**

| Assay of the immunosuppressive activity of IDO using acute rejection model (N=6) | | | |
|---|---|---|---|
| recipient | donor | treatment | average days of take (survival)(day) |
| LEW | DA | non-treated | 11.5 |
| LEW | DA | IDO protein (50 µg/rat) | 10.6 |
| LEW | DA | IDO protein (300 µg/rat) | >30 |

### (Example 8) Action of IDO inhibitor in sympatric liver transplantation

The sympatric liver transplantation model of a DA rat to a PVG rat for which a liver graft was known to be taken without the administration of an immunosuppresant was used to study the effect of the administration of an IDO inhibitor. As for the animals, male PVG and DA rats (200-350 g) were used. In the group administered with the IDO inhibitor, an Alza osmotic pressure pump (Muromachi Machinery Co., Ltd.) was filled with 2 ml of 1-methyl-tryptophan (IDO inhibitor: Arch. Biochem. Biophys., Vol. 291, 326, 1991, Aldrich Inc.), and immediately after the liver transplantation, it was embedded under the skin of the back by surgical operation.

As the results are shown in Table 4, there was a marked increase in the days of take (survival) for the group administered with the IDO inhibitor as compared to the group administered with physiological saline and to the non-treated group. Therefore, it was found that IDO was strongly involved in the take of an organ graft.

**Table 4**

| Action of IDO inhibitor in sympatric liver transplantation (N=6) | | | |
|---|---|---|---|
| recipient | donor | treatment | average days of take (survival)(day) |
| LEW | DA | non-treated | >60 |
| LEW | DA | physiological saline | >60 |
| LEW | DA | 1-methyl-tryptophan | 11.6 |

### (Preparation Example 2) Preparation of human IDO gene

The human IDO gene was prepared by PCR. Reaction was carried out similarly to Example 1, where 20 ng of Human Placenta cDNA Library (Takara Shuzo Co., Ltd.) was used as the template in combination with PCR primers (SEQ ID NO:11 and SEQ ID NO:12) (SAWADAY Technologies Co., Ltd.) and 40 cycles of PCR was conducted.

The solution after the reaction was subjected to electrophoresis using 1.0% agarose gel, and a PCR fragment of about 1.3 kb that had been specifically amplified was recovered from the gel with a Geneclean II kit (Funakoshi Co., Ltd.).

### (Example 9) Construction of human IDO expression plasmid

Plasmid (0.5 µg), pGEX 1 λ T (Amersham Pharmacia Biotech Inc.) and the recovered PCR fragment were treated with EcoRI (NEB) at 37 ° C overnight, respectively. By allowing to migrate by electrophoresis on 0.8% agarose gel, the respective fragments after the restriction enzyme treatment were recovered using a Geneclean II kit. These were mixed at the ratio of pGEX 1λT, 0.1 µg, to the PCR fragment, 0.5 µ g, and ligation reaction was carried out overnight using T4 DNA Ligase (GIBCO).

Competent cells of *E. coli* JM109 (Takara Shuzo Co., Ltd.), 100 µl, were dissolved in ice and to this was added 5 µl of the reaction solution after the ligation, which was allowed to stand in ice for 30 minutes. After incubation at 42 ° C for 90 seconds, the cells were placed in ice again for 5 minutes, and to this was added 900 µ 1 of SOC medium (GIBCO), followed by further incubation at 37 ° C for 1 hour. One hundred microliters was seeded on a LB agar medium containing 50 µg/ml of ampicillin (Wako Pure Chemical Industries, Ltd.) and incubated at 37 ° C overnight. Then, colonies were picked up and added to a LB liquid medium containing 50 µ g/ml of ampicillin; and further culturing was carried out at 37 ° C overnight. Purification of the plasmid was carried out using a Quiagen Plasmid MINI kit (QUIAGEN Corporation). The gene sequence of the obtained plasmid was analyzed with a DNA sequencer ALFexpress (Amersham Pharmacia Biotech Inc.) and was confirmed to be the normal IDO gene with no point mutation introduced. The foregoing manipulations produced an expression plasmid (IDO/pGEX) into which the human IDO gene as shown in SEQ ID NO:10 had been inserted.

### (Example 10) Purification of recombinant human IDO protein

After gene transfer to *E. coli* JM109 (Takara Shuzo Co., Ltd.) with the human IDO expression plasmid (IDO/pGEX) obtained in Example 9, isopropyl- β -D-thiogalactopyranoside (Wako Pure Chemical Industries, Ltd.) was added to produce a final concentration of 0.2 mM, whereby the expression of the recombinant IDO protein was induced. The purification of this protein and the measurement of its enzymatic activity were carried out according to the method of Maeda (Wakayama Med. Soc., Vol. 44, No. 3, 401, 1993). Finally, about 7.2 mg of the recombinant human IDO protein was obtained from 20 l of the *E. coli* culture solution.

### (Example 11) Studies on the immunosuppressive activity of human IDO protein using lymphocyte mixed culture test

To study the immunosuppressive activity of the human IDO protein prepared in Example 10, a lymphocyte mixed culture test was carried out. The spleen cells derived from a non-treated PVG rat and the spleen cells derived from a DA rat treated with 25 µg/ml of mitomycin (SIGMA-Aldrich Corporation) were seeded on a U bottom 96-well plate (NUNC Inc.) at 5x10⁴/well and at 8x10⁶/well, respectively. The IDO protein was then added and culturing was done for 4 days. Sixteen hours before the finish of culturing 5-bromo-2'deoxyuridine (BrdU) was added at a final concentration of 10 µM. The amount of BrdU incorporated into the intracellular DNA was quantified using a BrdU Labeling & Detection Kit III (Roche Diagnostics Inc.). As the results are shown in Fig. 2, the IDO protein showed the immunosuppressive activity in a concentration-dependent manner.

### Industrial Applicability

This invention relates to an immunosuppresant comprising the IDO gene and/or the IDO protein as the effective ingredient. According to this invention, single or repetitive administration of IDO is useful in the prevention of rejection in the tissue or organ transplantation. In addition, there are provided immunosuppresants effective as therapeutics for autoimmune diseases and inflammatory diseases such as allergic disorders.

## Claims

1. A gene therapeutic agent comprising a gene encoding indoleamine 2,3-dioxygenase (IDO).

2. The gene therapeutic agent comprising a gene encoding indoleamine 2,3-dioxygenase and a gene carrier.

3. The gene therapeutic agent according to claim 2, wherein the gene carrier is a viral vector.

4. The gene therapeutic agent according to claim 2, wherein the gene carrier is a cationic gene carrier.

5. The gene therapeutic agent according to claim 4, wherein the cationic gene carrier is either one selected from the group consisting of polydiaminobutyric acid and a pharmaceutically acceptable salt thereof.

6. The gene therapeutic agent according to claim 5, wherein the composition ratio of the gene encoding indoleamine 2,3-dioxygenase to the polydiaminobutyric acid or a pharmaceutically acceptable salt thereof is from 1/0.1 to 1/100 (w/w ratio).

7. An immunosuppresant comprising an indoleamine 2,3-dioxygenase protein as the effective ingredient.


SEQUENCE LISTING
<110>Hisamitsu Pharmaceutical Co., Inc

<120>Medical compound for gene therapy

<130>FP00-0333-00

<140>
<141>

<160> 12

<170> PatentIn Ver. 2.1

<210>1
<211>1294
<212>DNA
<213>mouse

<400>1
cattccttca gtggccaagt ggggggtcag tggagtagac agcaatggca ctcagtaaaa 60
tatctcctac agaaggttct agaaggatcc ttgaagacca ccacatagat gaagatgtgg 120
gctttgctct accacatcca ctggtggagc tgcccgacgc atacagcccc tgggtccttg 180
tggctagaaa tctgcctgtg ctgattgaga acgggcagct tcgagaagaa gttgaaaagc 240
tgcccacact gagcacggac ggactgagag gacacaggtt acagcgcctg gcacacctgg 300
ccctggggta catcaccatg gcgtatgtgt ggaaccgagg ggatgacgat gttcgaaagg 360
tgctgccccg caatattgct gttccctact gcgagctctc agagaagttg ggcctgcctc 420
ctattctgtc ttatgcagac tgtgtcctgg caaactggaa gaaaaaggac cccaatgggc 480
ccatgacata cgagaacatg gacattctgt tctcatttcc tggtggggac tgcgacaagg 540
gcttcttcct cgtctctcta ttggtggaaa tcgcagcttc tcctgcaatc aaagcaatcc 600
ccactgtatc cagtgcagta gagcgtcaag acctgaaagc attggaaaag gcactgcacg 660
acatagctac cagtctggag aaagccaagg aaatttttaa gaggatgcgt gactttgtgg 720
acccagacac gtttttccac gttctccgca tatatctgtc tggctggaaa tgcagctcca 780
agctgccaga aggtctgctg tatgaggggg tctgggacac cccaaaaatg ttttcagggg 840
gcagtgcagg ccagagcagc atcttccaga gtcttgatgt ccttctggga ataaaacacg 900
aggctggcaa agaatctcct gcagaattcc tccaggaaat gagagagtac atgcctccag 960

cccaccggaa cttccttttc ttcttagagt cagctccccc agtccgtgag tttgtcattt 1020
caagacacaa tgaagacttg acgaaagctt ataacgagtg tgtgaatggt ctggtctctg 1080
tgagaaagtt ccacctcgca atagtagata cttacattat gaaaccttcg aagaagaagc 1140
ccactgatgg cgacaagtcg gaagagccct caaatgtgga aagcagaggg actgggggta 1200
cgaatcccat gactttccta aggagtgtga aagatacaac cgagaaagct cttctgagtt 1260
ggccttagtg tagcaagctc cacttctatc aggg 1294

<210>2
<211>20
<212>DNA
<213>Artificial Sequence

<220>
<223>PCR primer

<400>2
gtacatcacc atggcgtatg 20

<210>3
<211>21
<212>DNA
<213>Artificial Sequence

<220>
<223>PCR primer

<400>3
gctttcgtca agtcttcatt g 21

<210>4
<211>29
<212>DNA
<213>Artificial Sequence

<220>
<223>PCR primer

<400>4
tcagctagcc attccttcag tggccaagt 29

<210>5
<211>29
<212>DNA
<213>Artificial Sequence

<220>
<223>PCR primer

<400>5
tgcctcgagc cctgatagaa gtggagctt 29

<210>6
<211>31
<212>DNA
<213>Artificial Sequence

<220>
<223>PCR primer

<400>6
tgcatttaaa tcattccttc agtggccaag t 31

<210>7
<211>31
<212>DNA
<213>Artificial Sequence

<220>
<223>PCR primer

<400>7
tcaatttaaa tccctgatag aagtggagct t 31

<210>8

<211>29

<212>DNA

<213>Artificial Sequence

<220>

<223>PCR primer

<400>8

tgccccggga tggcactcag taaaatatc 29

<210>9

<211>31

<212>DNA

<213>Artificial Sequence

<220>

<223>PCR primer

<400>9

tcagcggccg cctaaggcca actcagaaga g 31

<210>10

<211>1914

<212>DNA

<213>homo sapiens

<400>10

tgagaagggc aaatgctatc attggaaaaa ctgacaaaag tcccaatagg aaaaataagg 60

aagtggagag ttactatgtt tctaattttt catgtgcttc tatttttttc ctacttcaga 120

gccattgact aatagttgag tataacacag gttgtgtttc cgggctgctg aaacatgaca 180

ctaatatttt caaagaactg tggaagccta aaaggaagcc aatgagaaat aactaaatga 240

gagtttagga ctgcagcctt cattttcatt caaagattta aaagtttcca taaagtaaaa 300

tgttcttctc cggccacctg ttttcatagt tctgtgtttt ccttcaggcc tttctggctt 360

cctatatggc agtaagaaaa tgatgtgctt aatgattaca aatttcatat ggaatacgaa 420

ctttcagttt gtacatatga tgcacagaga tgcttttgtg gttttattgg ttttcatatt 480

acaaacaaag aaactagaaa atgaaaccat tccaaaagtg gaagtaattt ctcactgccc 540

ctgtgataaa ctgtggtcac tggctgtggc agcaactatt ataagatgct ctgaaactct 600
tcagacactg agggggcacc agaggagcag actacaagaa tggcacacgc tatggaaaac 660
tcctggacaa tcagtaaaga gtaccatatt gatgaagaag tgggctttgc tctgccaaat 720
ccacaggaaa atctacctga tttttataat gactggatgt tcattgctaa acatctgcct 780
gatctcatag agtctggcca gcttcgagaa agagttgaga agttaaacat gctcagcatt 840
gatcatctca cagaccacaa gtcacagcgc cttgcacgtc tagttctggg atgcatcacc 900
atggcatatg tgtggggcaa aggtcatgga gatgtccgta aggtcttgcc aagaaatatt 960
gctgttcctt actgccaact ctccaagaaa ctggaactgc ctcctatttt ggtttatgca 1020
gactgtgtct tggcaaactg gaagaaaaag gatcctaata agcccctgac ttatgagaac 1080
atggacgttt tgttctcatt tcgtgatgga gactgcagta aaggattctt cctggtctct 1140
ctattggtgg aaatagcagc tgcttctgca atcaaagtaa ttcctactgt attcaaggca 1200
atgcaaatgc aagaacggga cactttgcta aaggcgctgt tggaaatagc ttcttgcttg 1260
gagaaagccc ttcaagtgtt tcaccaaatc cacgatcatg tgaacccaaa agcatttttc 1320
agtgttcttc gcatatattt gtctggctgg aaaggcaacc cccagctatc agacggtctg 1380
gtgtatgaag ggttctggga agacccaaag gagtttgcag ggggcagtgc aggccaaagc 1440
agcgtctttc agtgctttga cgtcctgctg ggcatccagc agactgctgg tggaggacat 1500
gctgctcagt tcctccagga catgagaaga tatatgccac cagctcacag gaacttcctg 1560
tgctcattag agtcaaatcc ctcagtccgt gagtttgtcc tttcaaaagg tgatgctggc 1620
ctgcgggaag cttatgacgc ctgtgtgaaa gctctggtct ccctgaggag ctaccatctg 1680
caaatcgtga ctaagtacat cctgattcct gcaagccagc agccaaagga gaataagacc 1740
tctgaagacc cttcaaaact ggaagccaaa ggaactggag gcactgattt aatgaatttc 1800
ctgaagactg tgagaagtac aactgagaaa tcccttttga aggaaggtta atgtaaccca 1860
acaagagcac attttatcat agcagagaca tctgtatgca ttcctgtcat tacc 1914

<210>11
<211>28
<212>DNA
<213>Artificial Sequence

<220>
<223>PCR primer

<400>11
tcgaattcag aatggcacac gctatgga 28

<210>12
<211>21

<212>DNA
<213>Artificial Sequence

<220>
<223>PCR primer

<400>12
tcgaattcgc atacagatgt ctctgcta 28